(19) Europäisches Patentamt
European Patent Office
Office européen des brevets

(11) EP 3 273 236 B1

(12) **EUROPÄISCHE PATENTSCHRIFT**

(45) Veröffentlichungstag und Bekanntmachung des Hinweises auf die Patenterteilung:
**10.10.2018 Patentblatt 2018/41**

(51) Int Cl.:
***G01N 33/10*** *(2006.01)* ***A23L 5/20*** *(2016.01)*
***G01N 33/02*** *(2006.01)* ***G01N 1/40*** *(2006.01)*

(21) Anmeldenummer: **16001588.9**

(22) Anmeldetag: **18.07.2016**

(54) **VERFAHREN ZUR EXTRAKTION VON MYKOTOXINEN AUS GETREIDEN, ANDEREN LEBENSMITTELN UND FUTTERMITTELN**

METHOD FOR THE EXTRACTION OF MYCOTOXINS FROM CEREALS, OTHER FOOD ARTICLES AND FODDER

PROCEDE D'EXTRACTION DE MYCOTOXINES A PARTIR DE CEREALES, D'AUTRES PRODUITS ALIMENTAIRES ET ALIMENTS POUR ANIMAUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**24.01.2018 Patentblatt 2018/04**

(73) Patentinhaber: **R-Biopharm Aktiengesellschaft**
**64297 Darmstadt (DE)**

(72) Erfinder:
- **Winkle, Johannes (Ludwig Rudolf)**
  **69493 Hirschberg (DE)**
- **Blödorn, Dirk**
  **65931 Frankfurt am Main (DE)**
- **Zaid, Kholoud**
  **64283 Darmstadt (DE)**
- **Lacorn, Markus**
  **65375 Oestrich-Winkel (DE)**
- **Hektor, Thomas**
  **64347 Griesheim (DE)**

(74) Vertreter: **Baumbach, Friedrich**
**Patentanwalt**
**Robert-Rössle-Strasse 10**
**13125 Berlin (DE)**

(56) Entgegenhaltungen:
**WO-A1-2014/114982 WO-A1-2015/188205**
**US-A1- 2014 356 978**

- **HUYBRECHTS BART ET AL: "Méthodes analytiques de déterminationdes mycotoxines dans les produits agricoles :une revue", CAHIERS AGRICULTURES, Bd. 22, Nr. 3, Mai 2013 (2013-05), Seiten 202-215, XP002765170, DOI: 10.1684/agr.2013.0616**
- **SOLEIMANY F ET AL: "Determination of mycotoxins in cereals by liquid chromatography tandem mass spectrometry", FOOD CHEMISTRY, Bd. 130, Nr. 4, 31. Juli 2011 (2011-07-31) , Seiten 1055-1060, XP028294534, ELSEVIER LTD, NL ISSN: 0308-8146, DOI: 10.1016/J.FOODCHEM.2011.07.131 [gefunden am 2011-08-06]**
- **YIBADATIHAN S ET AL: "Simultaneous determination of multi-mycotoxins in palm kernel cake (PKC) using liquid chromatography-tandem mass spectrometry (LC-MS/MS)", FOOD ADDITIVES AND CONTAMINANTS PART A-CHEMISTRY ANALYSIS CONTROL EXPOSURE & RISK ASSESSMENT, Bd. 31, Nr. 12, Dezember 2014 (2014-12), Seiten 2071-2079, XP002765171,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 273 236 B1

## Beschreibung

### Gebiet der Erfindung

Die Erfindung betrifft ein Verfahren zur Extraktion von Mykotoxinen aus Getreiden und anderen Lebensmitteln oder aus Futtermitteln und deren anschließende Quantifizierung. Anwendungsgebiete sind die Lebensmittelindustrie, die Futtermittelindustrie oder die Biotechnologie.

### Hintergrund der Erfindung

Mykotoxine sind von Schimmelpilzen gebildete sekundäre Stoffwechselprodukte, die bei Wirbeltieren bereits in kleinsten Mengen giftig wirken. Derzeit sind etwa 200 verschiedene Mykotoxine bekannt, die von über 300 Pilzarten gebildet werden. Der Begriff Mykotoxine umfasst eine Reihe chemischer Verbindungen mit unterschiedlicher Struktur und Wirkung, die sich zu folgenden Stoffgruppen zusammenfassen lassen: Aflatoxine, Ochratoxine, Trichothecene wie beispielsweise Deoxynivalenol, Fumonisine, Alternaria-Toxine, Fusarium-Toxine, Mutterkornalkaloide.

Von besonderer Bedeutung in der Lebens- und Futtermittelindustrie sind Myktoxine der Aflatoxin-Gruppe, der Fumonisin-Gruppe, Ochratoxin A, Deoxynivalenol (DON), HT-2-Toxin (kurz HT2), T-2-Toxin (kurz T2) und Zearalenon. Aufgrund deren ausgesprochen hohen Gefährdungspotentials und der weiten Verbreitung können bereits kleinste Mengen in Lebens- und Futtermittel die Gesundheit von Mensch und Tier akut oder chronisch beeinträchtigen. Daher haben Gesetzgeber weltweit national gültige Grenzwerte für die verschiedenen Mykotoxine in unterschiedlichen Matrices erlassen. Als Beispiel sei an dieser Stelle die EU-Richtlinie 1881 aus dem Jahr 2006 genannt. Ferner wurden in diesem Zusammenhang Minimalanforderungen an Testsysteme (z.B. ELISA) in der EU durch die Richtlinie 519 aus dem Jahr 2014 festgelegt. Es besteht deshalb ein außerordentliches Interesse, Lebens- oder Futtermittel auf eine eventuelle Mykotoxinbelastung zu untersuchen. Als problematisch hat sich dabei in der Vergangenheit der Umstand erwiesen, dass die oben erwähnten Mykotoxine äußerst heterogen in ihrer Molekülstruktur sind und damit unterschiedlichste Eigenschaften wie z.B. unterschiedliche Lösungseigenschaften besitzen. So sind die hydrophoben Aflatoxine unlöslich in Wasser, während im Gegensatz dazu die Fumonisine und Deoxynivalenol in Wasser löslich sind (Fig. 1). Aflatoxine müssen daher unter Einsatz von Wasser-Methanol oder Wasser-Ethanol-Gemischen aus einer Probe extrahiert werden. Die anderen Mykotoxine wie z.B. T2 und HT2 (Fig. 1) nehmen hinsichtlich ihrer Polarität und Extrahierbarkeit eine Mittelstellung ein, benötigen aber auch Lösungsmittel wie Methanol zur Extraktion.

### Stand der Technik

Bisher erfolgte die Extraktion von Mykotoxinen unter Einsatz organischer Lösungsmittel wie Ethanol, Acetonitril oder Methanol. Allerdings fallen dabei große Mengen an organischen Lösungsmitteln an, die anschließend entsorgt werden müssen. Als Alternative dazu wurden Methoden entwickelt, bei denen durch Zusatz spezifischer Substanzen wie Cyclodextrine, proteinhaltige Komponenten (z.B. Rinderserumalbumin) bzw. Lösungsvermittler (z.B. nicht-ionische Tenside wie Triton oder Brij) bestimmte Mykotoxine extrahiert werden können. Solche Verfahren ohne organische Lösungsmittel sind häufig in den letzten Jahren patentiert worden (US 2014/0356978; WO 2015/188205; WO 2016/057044). Allerdings wird bei den Extraktionen ohne organische Lösungsmittel die Extraktion von Ochratoxin entweder nicht beschrieben oder erfordert wegen der im Molekül enthaltenen Carboxylfunktion den Einsatz eines speziellen Puffers (Mishra et al., 2016, Food Add.Contam. 33: 500-508). Eine Extraktionsmethode für alle relevanten Mykotoxine wurde noch nicht beschrieben.

Aufgrund der genannten Nachteile sind diese Methoden nicht Mittel der Wahl, um Lebensmittel wie beispielsweise Getreideproben schnell, einfach, umweltschonend und kostengünstig auf die Anwesenheit von verschiedensten Mykotoxinen zu analysieren.

### Ziel und Aufgabe der Erfindung

Die Erfindung hat das Ziel, verschiedenste Mykotoxine (Abb. 1) möglichst einheitlich mit einem einzigen Extraktionsmittel aus Lebensmitteln und Futtermitteln zu extrahieren.

Daraus leitet sich die Aufgabe der vorliegenden Erfindung ab, ein Extraktionsverfahren zu entwickeln, mit dem es möglich ist Mykotoxine mit unterschiedlichen Lösungseigenschaften einheitlich zu extrahieren

Insbesondere leitet sich daraus die Aufgabe ab, die Mykotoxine Aflatoxin, Deoxynivalenol, Ochratoxin A, Zearalenon, Fumonisin und T2/HT2 aus Getreide (Mais, Weizen, Roggen, Hafer, Gerste und deren Verwandte aus dem Tribus Triticeae), Futtermittel, Nüssen, Soja, Maisgluten, und Reis aber auch Feigen, Datteln, Rosinen und Pistazien zu extrahieren und anschließend zu quantifizieren.

Diese Aufgabe wird durch ein Verfahren gemäß Anspruch 1 gelöst. Weitere mögliche Ausführungsformen

ergeben sich aus den Unteransprüchen, der Beschreibung und den Beispielen.

Wesen der Erfindung

Überraschend wurde gefunden, dass mit Hilfe von wässrigen, gepufferten Naphthyl- und/oder Phenylverbindungen bzw. deren heterozyklische Analoga sowohl hydrophobe als auch hydrophile Mykotoxine extrahiert werden können.

Das erfindungsgemäße Verfahren ist dadurch gekennzeichnet, dass gepufferte Lösungen von Naphthyl- und/oder Phenylverbindungen und/oder deren heterozyklische Analoga, die wenigstens eine Sulfonsäure- bzw. wenigstens eine Carbonsäuregruppe tragen, mit den Getreiden oder anderen Lebensmitteln oder Futtermitteln in Kontakt gebracht werden, die wässrige Lösung anschließend abgetrennt und der Gehalt der extrahierten Mykotoxine in der wässrigen Lösung bestimmt wird.

Erfindungsgemäß werden Naphthyl- oder Phenylverbindungen oder deren heterozyklische Analoga der allgemeinen Formel I einzeln oder als Gemisch eingesetzt, um Mykotoxine verschiedenster Gruppen besonders effektiv zu extrahieren.

Formel I: 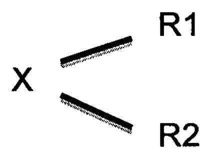

in der X, R1 und R2 folgende Bedeutung haben:

X = Naphthyl- oder Phenylrest oder deren heterozyklische Analoga
R1 = wenigstens eine Sulfonsäuregruppe oder wenigstens eine Carbonsäuregruppe
R2 = unsubstituiert oder eine funktionelle Gruppe ausgewählt aus Hydroxy-, Alkyl-, Alkoxy-, Amino-, Sulfhydryl-, Halogen- und Thioether, die in o-, m- oder p-Stellung zur Säuregruppe im Molekül angeordnet sind, wobei α-Aminosäuren ausgeschlossen sind.

Nachfolgend sind Beispiele aufgelistet, die in der Lage sind Mykotoxine verschiedenster Gruppen zu extrahieren:

1,5-Naphthyldisulfonsäure
2,6-Naphthyldisulfonsäure
4-Hydroxyphenylsulfonsäure
Benzolsulfonsäure,
4-Methylbenzolsulfonsäure,
Benzol-1,3-disulfonsäure,
1-Naphthol-3,6-disulfonsäure,
3-Sulfobenzoesäure,
4-Sulfobenzoesäure,
2-Hydroxybenzoesäure,
2,6-Dihydroxybenzoesäure,
2,5-Dihydroxybenzoesäure,
2,4-Dihydroxybenzoesäure,
3,4-Dihydroxybenzoesäure,
3,5-Dihydroxybenzoesäure.
2-Hydroxy-5-sulfobenzoesäure

Als besonders vorteilhaft stellte sich der alleinige aber auch kombinierte Einsatz von 1,5-Naphtyldisulfonsäure, 2,6-Naphtyldisulfonsäure und/oder p-Hydroxyphenylsulfonsäure heraus.

Der resultierende wässrige Überstand, der die extrahierten Mykotoxine enthält, wird anschließend abgetrennt und für die Analytik eingesetzt. So kann die Bestimmung oder die weitere Reinigung der Mykotoxine beispielsweise mit Hilfe von enzymatischen, enzymimmunologischen, chromatographisch-gestützten und/oder immunoaffinitätschromatographischen Verfahren erfolgen.

Für alle nachfolgend beschriebene Versuche kommen zertifizierte Referenzmaterialien zum Einsatz (Tabelle 1). Für jedes Mykotoxin werden eine Blankprobe und eine kontaminierte Referenzprobe vermessen.

Tabelle 1. Zertifizierte Mykotoxin-Referenzmaterialien zur Überprüfung des Extraktionseffizienz der beanspruchten Verfahren

| Mykotoxin (Matrix) | Referenzwert* | Zertifiziertes Material Bezeichnung** |
|---|---|---|
| Aflatoxine (Mais) | n.d. | AC 215 |
| | 31,2 ± 3,1 μg/kg | AC 295 |
| Ochratoxin (Mais) | n.d. | OC 853 |
| | 12,3 ± 1,3 μg/kg | OC 866 |
| Deoxynivalenol (Weizen) | n.d. | DW 100 |
| | 2,1 ± 0,3 mg/kg | DW 174 |
| Fumonisine (Mais) | n.d. | FC 400 |
| | 0,5 ± 0,07 mg/kg | FC 458 |
| Zearalenon (Mais) | n.d. | ZC 300 |
| | 472,1 ± 65,6 μg/kg | ZC 321 |
| T2/HT2 (Mais) | n.d. | TC 978 |
| | 255,7 ± 18 μg/kg T2 und 681,1 ± 45,8 μg/kg HT2 | TC 982 |

*nicht detektiert mittels HPLC; siehe Zertifikate der Firma Trilogy für nähere Erläuterungen wie z.B. Nachweisgrenze der verwendeten Methode
**zum Einsatz kamen Materialien der Firma Trilogy (Washington, MO, USA)

Die Erfindung wird nachfolgend an der Bestimmung von Aflatoxin in Mais näher erläutert. Die Referenz-Extraktionsmethode sieht eine Probeneinwaage von 1 g vor, welche mit 5 mL 70% Methanol in Wasser 10 min unter Schütteln extrahiert wird (Tabelle 2). Nach Zentrifugation oder Filtration wird der Überstand 1:7 mit destilliertem Wasser verdünnt (z.B. 100 μL Extrakt + 600 μL Wasser) und in einem handelsüblichen kommerziellen ELISA der Aflatoxingehalt quantifiziert. Zum Einsatz kommt der RIDASCREEN® Aflatoxin Total (Art. Nr. 4701, R-Biopharm AG, Darmstadt, siehe auch Tabelle 2). Grundlage ist die Antigen-Antikörper-Reaktion. Die Vertiefungen der Mikrotiterplatten sind mit Fänger-Antikörpern gegen anti-Aflatoxin-Antikörper beschichtet. Zugegeben werden Kalibratoren bzw. extrahierte Probenlösung, enzymmarkiertes Aflatoxin (Enzymkonjugat) und anti-Aflatoxin-Antikörper. Freies und enzymmarkiertes Aflatoxin konkurrieren um die Aflatoxin-Antikörperbindungsstellen. Gleichzeitig werden auch die anti-Aflatoxin-Antikörper von den immobilisierten Fänger-Antikörpern gebunden. Nicht gebundenes enzymmarkiertes Aflatoxin wird anschließend in einem Waschschritt wieder entfernt. Der Nachweis erfolgt durch Zugabe von Substrat-/Chromogenlösung. Gebundenes Enzymkonjugat wandelt das Chromogen in ein blaues Endprodukt um. Die Zugabe des Stopp-Reagenzes führt zu einem Farbumschlag von blau nach gelb. Die Messung erfolgt photometrisch bei 450 nm; die gemessene optische Dichte (OD)der Lösung ist umgekehrt proportional zur Aflatoxin-Konzentration in der Probe.

Tabelle 2. Angewandte Referenzextraktion und eingesetztes ELISA-System zum Vergleich der Extraktionseffizienz der beanspruchten Verfahren

| Mykotoxin (Matrix) | Referenzextraktion | ELISA |
|---|---|---|
| Aflatoxine (Mais) | 1 g Probe + 5 mL 70% Methanol in Wasser; Verdünnung 1:7 mit Wasser | R4701 (RIDASCREEN® Aflatoxin Total) |
| Ochratoxin (Mais) | 1 g Probe + 5 mL 130 mM $NaHCO_3$ (pH 8.1); Verdünnung 1:4 mit Wasser | R1311 (RIDASCREEN® Ochratoxin A 30/15 |
| Deoxynivalenol (Weizen) | 1 g Probe + 5 mL Wasser; Verdünnung 1: 10 mit Wasser | R5906 (RIDASCREEN® DON) |

(fortgesetzt)

| Mykotoxin (Matrix) | Referenzextraktion | ELISA |
|---|---|---|
| Fumonisine (Mais) | 1 g Probe + 5 mL 70% Methanol in Wasser; Verdünnung 1:14 mit Wasser | R3401 (RIDASCREEN® Fumonisin) |
| Zearalenon (Mais) | 1 g Probe + 5 mL 70% Methanol in Wasser; Verdünnung mit Puffer | R1401 (RIDASCREEN® Zearalenon) |
| T2/HT2 (Mais) | 1 g Probe + 5 mL 70% Methanol in Wasser; Verdünnung 1:20 mit Wasser | R3805 (RIDASCREEN® T2 / HT2 Toxin |

Nachfolgend ist als Beispiel ein bevorzugtes Verfahren für die Extraktion von Aflatoxin aus Mais dargestellt. Dazu wird 1 g homogenisierte Maisprobe mit 5 mL einer Lösung bestehend aus 250 mM 1,5-Naphthyldisulfonsäure gepuffert auf pH 8 mit 100 mM Tris-(hydroxymethyl)-aminomethan (Tris) versetzt, 10 min geschüttelt und zentrifugiert bzw. filtriert. Der klare Überstand wird 1:7 mit destilliertem Wasser verdünnt und wie oben bereits beschrieben im ELISA vermessen.

Die Ergebnisse sind in Tabelle 3 dargestellt. Dabei ist zu beachten, dass aus Vergleichsbarkeitsgründen die Ergebnisse als "Signalreduktion in %" angegeben werden. Die Signalreduktion ergibt sich aus dem kompetitiven Format des ELISAs als Testsystems. Bewertet werden soll die Extraktionsausbeute im Vergleich zwischen dem neuen, beanspruchten Verfahren und der etablierten Referenzextraktion. Eine hohe Analytkonzentration im Extrakt (bedingt durch eine hohe Extraktionsausbeute) führt zu einer Reduktion des Messsignals ($OD_{450\ nm}$) Die Signalreduktion in [%] berechnet sich wie folgt:

$$\text{Signalreduktion [\%]} = 100 - (OD_{\text{positive Probe}}/OD_{\text{negative Probe}} \times 100)$$

Tabelle 3: Extraktionsausbeute von Aflatoxin aus Mais unter Einsatz der beanspruchten Substanz 1,5-Naphthyldisulfonsäure (in Tris-Puffer pH 8.0) im Vergleich zur Referenzextraktion. Die OD-Werte wurde im kommerziell erhältlichen ELISA RIDASCREEN® Aflatoxin Total ermittelt.

| | Referenzextraktion | 250 mM 1,5-NDS + 100 mM Tris pH 8.0 |
|---|---|---|
| $OD_{Blank}$ | 1,591 | 1,966 |
| $OD_{Positiv}$ | 0,474 | 0,525 |
| Signalreduktion [%] | 71 | 73 |

Vergleicht man die Werte der Referenzextraktion mit denen der beanspruchten Extraktion, so wird deutlich, dass diese sehr ähnlich sind. Aflatoxin wurde somit erfolgreich aus der Matrix Mais durch das beanspruchte Verfahren quantitativ extrahiert. Geringe Unterschiede in den OD-Werten vor allem der Blankproben können durch Sekundäreffekte der beanspruchten Substanzen im Messsystem ELISA erklärt werden. Durch Wechselwirkungen wird die Kompetition zwischen Aflatoxin aus dem Extrakt (Probe) und Aflatoxin-Enzym-Konjugat um Antikörperbindungsstellen leicht gestört. Dieses ist auch der Grund, warum keine Konzentrationswerte ermittelt worden sind, sondern die Ergebnisse als "Signalreduktion [%]" angegeben werden. Diese würden ein falsches Bild entstehen lassen, weil die Beeinflussung des ELISAs nicht Teil der beanspruchten Extraktion ist. Die ELISA Systeme können entsprechend auf solche Substanzen eingestellt werden, so dass die Richtigkeit des Messsystems gegeben ist.

Unabhängig von dem oben aufgeführten Beispiel ist es aber auch möglich, andere Proben aus dem Lebensmittel- bzw. Futtermittelbereich zu untersuchen wie beispielsweise Weizen, Roggen, Hafer, Gerste und deren Verwandte aus dem Tribus Triticeae, Futtermittel, Nüssen, Soja, Maisgluten und Reis. Ferner sind Feigen, Datteln, Rosinen und Pistazien von Interesse.

Ebenso ist die Extraktion nicht auf die oben genannten Aflatoxine limitiert. So ist es auch möglich, dass Deoxynivalenol, Ochratoxin A, Zearalenon, Fumonisin und T2/HT2 mit dem erfindungsgemäßen Verfahren extrahiert werden. Ebenso können andere Toxine wie Ergotalkaloide, Citrinin, und Sterigmatocystin mit dem erfindungsgemäßen Verfahren extrahiert werden.

Untersuchungen haben gezeigt, dass die Effektivität des Verfahrens durch ein geeignetes Puffersystem positiv beeinflusst wird. So sollte die wässrige Lösung der Naphthyl- oder Phenylverbindungen oder deren heterozyklische

Analoga im Bereich von pH 5 - 10 gepuffert vorliegen. Dabei sind Tris-(hydroxymethyl)-aminomethan (Tris)- und "Imidazol"-haltige Puffer im Bereich von pH 7.5 - 8.5 als bevorzugt anzusehen (siehe Beispiel in Tablle 3). Der "Imidazol"-Puffer wurde durch Mischung von Isopropylimidazol und Imidazolhydrochlorid in den entsprechenden Anteilen angesetzt. Andere Puffersysteme wie Phosphat und EPPS (N-(2-Hydroxyethyl)-piperazin-N'-(3-propansulfonsäure) sind ebenfalls möglich, können aber in Abhängigkeit von Konzentration und Mykotoxin abweichende Extraktionsergebnisse aufweisen.

Möglich ist auch ein Extraktionsverfahren, bei dem aromatische Phenyl- oder Naphthylverbindungen verwendet werden, mit wenigstens einer mit Stickstoff substituierten Position.

Das Verfahren weist gegenüber den bisher bekannten Extraktionsverfahren den Vorteil auf, dass alle wichtigen Mykotoxine mit einem umweltschonenden, wässrigen Extraktionsmittel effizient und möglichst vollständig aus relevanten Lebensmittelmatrices extrahiert werden können. Die Analyse mehrerer unterschiedlicher Mykotoxine erfordert nicht mehr, wie in den herkömmlichen Verfahren, separate Probeneinwaagen und individuelle Extraktionsmittel, sondern lediglich eine einmalige Probeneinwaage und eine universelle Extraktion mit dem beanspruchten Verfahren. Dadurch ergeben sich für den Anwender dieses neuen Verfahrens eine signifikante Zeit- und Materialersparnis, die zu einer Kostenreduktion führt sowie die Vermeidung einer Exposition mit gesundheitsgefährdenden Lösungsmitteln aus den Extraktionsmitteln herkömmlicher Verfahren.

Die Erfindung wird nachfolgend mit Ausführungsbeispielen näher erläutert.

## Ausführungsbeispiele

### Beispiel 1

Tabelle 4: Extraktionsausbeute von Aflatoxin aus Mais unter Einsatz der beanspruchten Substanzen 1,5-Naphtyldisulfonsäure (1,5-NDS, 250 mM) unter Einsatz verschiedener Puffer (100 mM) und pH-Werte im Vergleich zur Referenzextraktion (Ref., siehe auch Tabelle 2). Die OD-Werte wurde im kommerziell erhältlichen ELISA RIDASCREEN® Aflatoxin Total ermittelt.

| | Ref. | 1,5-NDS Tris pH 8.0 | 1,5-NDS Tris pH 8.5 | 1,5-NDS Imidazol pH 8.0 | 1,5-NDS Imidazol pH 8.5 |
|---|---|---|---|---|---|
| $OD_{Blank}$ | 1,591 | 1,966 | 1,917 | 1,684 | 1,578 |
| $OD_{Positiv}$ | 0,474 | 0,525 | 0,491 | 0,401 | 0,326 |
| Signalreduktion [%] | 71 | 73 | 74 | 76 | 79 |

### Beispiel 2

Tabelle 5: Extraktionsausbeute von Aflatoxin aus Mais unter Einsatz der beanspruchten Substanzen 2,6-Naphtyldisulfonsäure (2,6-NDS, 125 mM) unter Einsatz verschiedener Puffer (100 mM) und pH-Werte im Vergleich zur Referenzextraktion (Ref., siehe auch Tabelle 2). Die OD-Werte wurde im kommerziell erhältlichen ELISA RIDASCREEN® Aflatoxin Total ermittelt.

| | Ref. | 2,6-NDS Tris pH 8.0 | 2,6-NDS Tris pH 8.5 | 2,6-NDS Imidazol pH 8.0 | 2,6-NDS Imidazol pH 8.5 |
|---|---|---|---|---|---|
| $OD_{Blank}$ | 1,505 | 1,686 | 1,711 | 1,654 | 1,645 |
| $OD_{Positiv}$ | 0,377 | 0,435 | 0,356 | 0,403 | 0,393 |
| Signalreduktion [%] | 75 | 74 | 79 | 76 | 76 |

### Beispiel 3

Tabelle 6: Extraktionsausbeute von DON aus Weizen unter Einsatz der beanspruchten Substanzen 1,5-Naphtyldisulfonsäure (1,5-NDS, 250 mM) unter Einsatz verschiedener Puffer (100 mM) und pH-Werte im Vergleich zur Referenzextraktion (Ref., siehe auch Tabelle 2). Die OD-Werte wurde im kommerziell erhältlichen ELISA RIDASCREEN® DON ermittelt.

| | Ref. | 1,5-NDS Tris pH 8.0 | 1,5-NDS Tris pH 8.5 | 1,5-NDS Imidazol pH 8.0 | 1,5-NDS Imidazol pH 8.5 |
|---|---|---|---|---|---|
| $OD_{Blank}$ | 2,440 | 2,682 | 2,722 | 2,649 | 2,638 |
| $OD_{Positiv}$ | 0,738 | 0,795 | 0,842 | 0,744 | 0,811 |
| Signalreduktion [%] | 70 | 70 | 69 | 72 | 69 |

Beispiel 4

Tabelle 7: Extraktionsausbeute von DON aus Weizen unter Einsatz der beanspruchten Substanzen 2,6-Naphtyldisulfonsäure (2,6-NDS, 125 mM) unter Einsatz verschiedener Puffer (100 mM) und pH-Werte im Vergleich zur Referenzextraktion (Ref., siehe auch Tabelle 2). Die OD-Werte wurde im kommerziell erhältlichen ELISA RIDASCREEN® DON ermittelt.

| | Ref. | 2,6-NDS Tris pH 8.0 | 2,6-NDS Tris pH 8.5 | 2,6-NDS Imidazol pH 8.0 | 2,6-NDS Imidazol pH 8.5 |
|---|---|---|---|---|---|
| $OD_{Blank}$ | 2,463 | 2,694 | 2,667 | 2,610 | 2,657 |
| $OD_{Positiv}$ | 0,886 | 0,923 | 0,923 | 0,871 | 0,906 |
| Signalreduktion [%] | 64 | 66 | 65 | 67 | 66 |

Beispiel 5

Tabelle 8: Extraktionsausbeute von Ochratoxin A aus Mais unter Einsatz der beanspruchten Substanzen 1,5-Naphtyldisulfonsäure (1,5-NDS, 250 mM) unter Einsatz verschiedener Puffer (100 mM) und pH-Werte im Vergleich zur Referenzextraktion (Ref., siehe auch Tabelle 2). Die OD-Werte wurde im kommerziell erhältlichen ELISA RIDASCREEN® Ochratoxin A 30/15 ermittelt.

| | Ref. | 1,5-NDS Tris pH 8.0 | 1,5-NDS Tris pH 8.5 | 1,5-NDS Imidazol pH 8.0 | 1,5-NDS Imidazol pH 8.5 |
|---|---|---|---|---|---|
| $OD_{Blank}$ | 1,551 | 1,447 | 1,374 | 1,479 | 1,277 |
| $OD_{Positiv}$ | 0,305 | 0,338 | 0,306 | 0,366 | 0,296 |
| Signalreduktion [%] | 80 | 77 | 78 | 75 | 77 |

Beispiel 6

Tabelle 9: Extraktionsausbeute von Ochratoxin A aus Mais unter Einsatz der beanspruchten Substanzen 2,6-Naphtyldisulfonsäure (2,6-NDS, 125 mM) unter Einsatz verschiedener Puffer (100 mM) und pH-Werte im Vergleich zur Referenzextraktion (Ref., siehe auch Tabelle 2). Die OD-Werte wurde im kommerziell erhältlichen ELISA RIDASCREEN® Ochratoxin A 30/15 ermittelt.

| | Ref. | 2,6-NDS Tris pH 8.0 | 2,6-NDS Tris pH 8.5 | 2,6-NDS Imidazol pH 8.0 | 2,6-NDS Imidazol pH 8.5 |
|---|---|---|---|---|---|
| $OD_{Blank}$ | 1,551 | 1,602 | 1,462 | 1,425 | 1,374 |

(fortgesetzt)

| | Ref. | 2,6-NDS Tris pH 8.0 | 2,6-NDS Tris pH 8.5 | 2,6-NDS Imidazol pH 8.0 | 2,6-NDS Imidazol pH 8.5 |
|---|---|---|---|---|---|
| $OD_{Positiv}$ | 0,305 | 0,407 | 0,309 | 0,324 | 0,263 |
| Signalreduktion [%] | 80 | 75 | 79 | 77 | 81 |

Beispiel 7

Tabelle 10: Extraktionsausbeute von Zearalenon aus Mais unter Einsatz der beanspruchten Substanzen 1,5-Naphtyldisulfonsäure (1,5-NDS, 250 mM) oder 2,6-Naphthyldisulfonsäure unter Einsatz verschiedener Puffer (2,6-NDS, 100 mM) und pH-Werte im Vergleich zur Referenzextraktion (Ref., siehe auch Tabelle 2). Die OD-Werte wurde im kommerziell erhältlichen ELISA RIDASCREEN® Zearalenon ermittelt.

| | Ref. | 1,5-NDS Tris pH 8.0 | 1,5-NDS Imidazol pH 8.5 | 2,6-NDS Tris pH 8.0 | 2,6-NDS Imidazol pH 8.0 |
|---|---|---|---|---|---|
| $OD_{Blank}$ | 3,093 | 2,594 | 2,540 | 2,403 | 2,214 |
| $OD_{Positiv}$ | 0,227 | 0,355 | 0,268 | 0,245 | 0,301 |
| Signalreduktion [%] | 93 | 86 | 89 | 90 | 86 |

Beispiel 8

Tabelle 11: Extraktionsausbeute von Fumonisin aus Mais unter Einsatz der beanspruchten Substanzen 1,5-Naphtyldisulfonsäure (1,5-NDS, 50 mM) oder 2,6-Naphthyldisulfonsäure unter Einsatz verschiedener Puffer (2,6-NDS, 100 mM) und pH-Werte im Vergleich zur Referenzextraktion (Ref., siehe auch Tabelle 2). Die OD-Werte wurde im kommerziell erhältlichen ELISA RIDASCREEN® Fumonisin ermittelt.

| | Ref. | 1,5-NDS Tris pH 8.0 | 1,5-NDS Imidazol pH 8.0 | 2,6-NDS Tris pH 8.0 | 2,6-NDS Imidazol pH 8.0 |
|---|---|---|---|---|---|
| $OD_{Blank}$ | 1,193 | 0,719 | 0,762 | 0,874 | 0,881 |
| $OD_{Positiv}$ | 0,339 | 0,174 | 0,151 | 0,202 | 0,188 |
| Signalreduktion [%] | 72 | 76 | 80 | 77 | 79 |

Beispiel 9

Tabelle 12: Extraktionsausbeute von T2 und HT2 aus Mais unter Einsatz der beanspruchten Substanzen 1,5-Naphtyldisulfonsäure (1,5-NDS, 250 mM) oder 2,6-Naphthyldisulfonsäure unter Einsatz verschiedener Puffer (2,6-NDS, 100 mM) und pH-Werte im Vergleich zur Referenzextraktion (Ref., siehe auch Tabelle 2). Die OD-Werte wurde im kommerziell erhältlichen ELISA RIDASCREEN® T2/HT2 ermittelt.

| | Ref. | 1,5-NDS Tris pH 8.0 | 1,5-NDS Imidazol pH 8.0 | 2,6-NDS Tris pH 8.0 | 2,6-NDS Imidazol pH 8.0 |
|---|---|---|---|---|---|
| $OD_{Blank}$ | 1,676 | 0,947 | 0,921 | 0,868 | 0,799 |
| $OD_{Positiv}$ | 0,467 | 0,289 | 0,282 | 0,270 | 0,265 |
| Signalreduktion [%] | 72 | 69 | 69 | 69 | 67 |

Beispiel 10

Tabelle 13: Extraktionsausbeute von Aflatoxin aus Mais unter Einsatz der beanspruchten Substanzen 1,5-Naphtyldisulfonsäure (1,5-NDS, 250 mM) oder 2,6-Naphthyldisulfonsäure (2,6-NDS, 100 mM) unter Einsatz verschiedener Pufferstärken und pH-Werte im Vergleich zur Referenzextraktion (Ref., siehe auch Tabelle 2). Die OD-Werte wurde im kommerziell erhältlichen ELISA RIDASCREEN® Aflatoxin Total ermittelt.

| | Ref. | 1,5-NDS Phosphat 200 mM pH 8.0 | 1,5-NDS Epps* 100 mM pH 8.5 | 2,6-NDS Phosphat 25 mM pH 8.0 | 2,6-NDS Phosphat 75 mM pH 8.5 |
|---|---|---|---|---|---|
| $OD_{Blank}$ | 1,487 | 1,542 | 1,607 | 1,474 | 1,445 |
| $OD_{Positiv}$ | 0,326 | 0,303 | 0,350 | 0,339 | 0,350 |
| Signalreduktion [%] | 78 | 80 | 78 | 77 | 76 |

*(N-(2-hydroxyethyl)-piperazin-N'-(3-propansulfonsäure)

Beispiel 11

Tabelle 14: Extraktionsausbeute von Aflatoxin aus Mais unter Einsatz der beanspruchten Substanz 1,5-Naphtyldisulfonsäure in verschiedenen Konzentrationen bei pH 8.0 (5 mM Phosphat) im Vergleich zur Referenzextraktion (Ref., siehe auch Tabelle 2). Die OD-Werte wurde im kommerziell erhältlichen ELISA RIDASCREEN® Aflatoxin Total ermittelt.

| | Ref. | 300 mM | 200 mM | 100 mM | 50 mM | 10 mM |
|---|---|---|---|---|---|---|
| $OD_{Blank}$ | 1,320 | 1,516 | 1,611 | 1,601 | 1,596 | 1,601 |
| $OD_{Positiv}$ | 0,325 | 0,309 | 0,373 | 0,452 | 0,752 | 0,920 |
| Signalreduktion [%] | 75 | 80 | 77 | 72 | 53 | 43 |

Beispiel 12

Tabelle 15: Extraktionsausbeute von Aflatoxin aus Mais unter Einsatz der beanspruchten Substanz 2,6-Naphtyldisulfonsäure in verschiedenen Konzentrationen bei pH 8.0 (5 mM Phosphat) im Vergleich zur Referenzextraktion (Ref., siehe auch Tabelle 2). Die OD-Werte wurde im kommerziell erhältlichen ELISA RIDASCREEN® Aflatoxin Total ermittelt.

| | Ref. | 100 mM | 75 mM | 50 mM | 10 mM | 5 mM |
|---|---|---|---|---|---|---|
| $OD_{Blank}$ | 1,544 | 1,860 | 1,854 | 1,881 | 1,962 | 1,973 |
| $OD_{Positiv}$ | 0,438 | 0,489 | 0,521 | 0,620 | 1,101 | 1,051 |
| Signalreduktion [%] | 72 | 74 | 72 | 67 | 44 | 47 |

Beispiel 13

Tabelle 16: Extraktionsausbeute von Deoxynivalenol, Fumonisin und Zearalenon (Matrices siehe Tabelle 1) unter Einsatz der beanspruchten Substanz 4-Hydroxyphenylsulfonsäure (375 mM; 4-OH-PSN) unter Zusatz von 50 mM Phosphat (pH 8.0) im Vergleich zur Referenzextraktion (Ref., siehe auch Tabelle 2). Die OD-Werte wurde in kommerziell erhältlichen ELISA der RIDASCREEN® Serie ermittelt (siehe Tabelle 2).

| | DON Ref. | DON 4-OH-PSN | Fumo* Ref. | Fumo* 4-OH-PSN | Zea* Ref. | Zea* 4-OH-PSN |
|---|---|---|---|---|---|---|
| $OD_{Blank}$ | 2,337 | 2,577 | 1,261 | 0,814 | 3,012 | 2,338 |
| $OD_{Positiv}$ | 0,786 | 0,824 | 0,398 | 0,195 | 0,195 | 0,294 |
| Signalreduktion [%] | 66 | 68 | 68 | 76 | 94 | 87 |

*DON, Deoxynivalenol; Fumo, Fumonisin; Zea, Zearalenon

Beispiel 14

Tabelle 17: Extraktionsausbeute von Aflatoxin, Ochratoxin und T2/HT2 (Matrices siehe Tabelle 1) unter Einsatz der beanspruchten Substanz 4-Hydroxyphenylsulfonsäure (375 mM; 4-OH-PSN) unter Zusatz von 50 mM Phosphat (pH 8.0) im Vergleich zur Referenzextraktion (Ref., siehe auch Tabelle 2). Die OD-Werte wurde in kommerziell erhältlichen ELISA der RIDASCREEN® Serie ermittelt (siehe Tabelle 2).

| | Afla* Ref. | Afla* 4-OH-PSN | OTA* Ref. | OTA* 4-OH-PSN | T2/HT2 Ref. | T2/HT2 4-OH-PSN |
|---|---|---|---|---|---|---|
| $OD_{Blank}$ | 1,701 | 1,821 | 1,551 | 1,557 | 1,889 | 0,965 |
| $OD_{Positiv}$ | 0,451 | 0,673 | 0,305 | 0,308 | 0,525 | 0,342 |
| Signalreduktion [%] | 73 | 63 | 80 | 80 | 72 | 65 |

*Afla, Aflatoxin; OTA, Ochratoxin

Beispiel 15

Tabelle 18: Extraktionsausbeute von Aflatoxin aus Mais unter Einsatz der beanspruchten Substanz 4-Hydroxyphenylsulfonsäure in verschiedenen Konzentrationen bei pH 8.0 (5 mM Phosphat) im Vergleich zur Referenzextraktion (Ref., siehe auch Tabelle 2). Die OD-Werte wurde im kommerziell erhältlichen ELISA RIDASCREEN® Aflatoxin Total ermittelt.

| | Ref. | 600 mM | 500 mM | 400 mM | 300 mM | 200 mM | 100 mM |
|---|---|---|---|---|---|---|---|
| $OD_{Blank}$ | 1,485 | 1,692 | 1,707 | 1,712 | 1,722 | 1,692 | 1,649 |
| $OD_{Positiv}$ | 0,440 | 0,512 | 0,533 | 0,547 | 0,568 | 0,676 | 0,855 |
| Signalreduktion [%] | 70 | 70 | 69 | 68 | 67 | 60 | 48 |

Beispiel 16

Tabelle 19: Extraktionsausbeute von Ochratoxin aus Mais unter Einsatz der beanspruchten Substanz 4-Hydroxyphenylsulfonsäure in verschiedenen Konzentrationen bei pH 8.0 (5 mM Phosphat) im Vergleich zur Referenzextraktion (Ref., siehe auch Tabelle 2). Die OD-Werte wurde im kommerziell erhältlichen ELISA RIDASCREEN® Ochratoxin A 30/15 ermittelt.

| | Ref. | 600 mM | 500 mM | 400 mM | 300 mM | 200 mM | 50 mM |
|---|---|---|---|---|---|---|---|
| $OD_{Blank}$ | 1,620 | 1,308 | 1,365 | 1,345 | 1,383 | 1,475 | 1,692 |
| $OD_{Positiv}$ | 0,239 | 0,264 | 0,229 | 0,264 | 0,241 | 0,267 | 0,309 |
| Signalreduktion [%] | 85 | 80 | 83 | 80 | 83 | 82 | 82 |

Beispiel 17

Tabelle 20: Extraktionsausbeute von Ochratoxin aus Mais unter kombiniertem Einsatz der beanspruchten Substanzen 1,5-Naphthyldisulfonsäure (1,5-NDS, 250 mM) und 4-Hydroxyphenylsulfonsäure (verschiedene Konzentrationen von 10 mM bis 150 mM) bei pH 8.0 (5 mM Phosphat) im Vergleich zur Referenzextraktion (Ref., siehe auch Tabelle 2). Die OD-Werte wurde im kommerziell erhältlichen ELISA RIDASCREEN® Ochratoxin A 30/15 ermittelt.

| | | 250 mM 1,5-NDS | | | | | |
|---|---|---|---|---|---|---|---|
| | Ref. | 150 mM | 100 mM | 75 mM | 50 mM | 20 mM | 10 mM |
| $OD_{Blank}$ | 1,551 | 1,120 | 1,231 | 1,360 | 1,352 | 1,494 | 1,495 |
| $OD_{Positiv}$ | 0,305 | 0,330 | 0,375 | 0,321 | 0,355 | 0,422 | 0,485 |
| Signalreduktion [%] | 80 | 71 | 70 | 76 | 74 | 72 | 68 |

Beispiel 18

Tabelle 21: Extraktionsausbeute von Ochratoxin aus Mais unter kombiniertem Einsatz der beanspruchten Substanzen 1,5-Naphthyldisulfonsäure (1,5-NDS, 250 mM) und 4-Hydroxyphenylsulfonsäure (4-OH-PSN, 50 mM und 75 mM) bei verschiedenen pH-Werten von 7.5 bis 9.0 im Vergleich zur Referenzextraktion (Ref., siehe auch Tabelle 2). Die OD-Werte wurde im kommerziell erhältlichen ELISA RIDASCREEN® Ochratoxin A 30/15 ermittelt.

| | 250 mM 1,5-NDS | | | | | | | | |
|---|---|---|---|---|---|---|---|---|---|
| | | 50 mM 4-OH-PSN | | | | 75 mM 4-OH-PSN | | | |
| | Ref. | pH 7.5 | pH 8.0 | pH 8.5 | pH 9.0 | pH 7.5 | pH 8.0 | pH 8.5 | pH 9.0 |
| $OD_{Blank}$ | 1,551 | 1,342 | 1,249 | 1,210 | 1,265 | 1,244 | 1,223 | 1,139 | 1,108 |
| $OD_{Positiv}$ | 0,305 | 0,348 | 0,292 | 0,205 | 0,240 | 0,379 | 0,358 | 0,278 | 0,257 |
| Signalreduktion [%] | 80 | 74 | 77 | 83 | 81 | 70 | 71 | 76 | 77 |

Beispiel 19

Tabelle 22: Extraktionsausbeute von Ochratoxin aus Mais unter kombiniertem Einsatz der beanspruchten Substanzen 2,6-Naphthyldisulfonsäure (125 mM) und 4-Hydroxyphenylsulfonsäure (verschiedene Konzentrationen von 20 mM bis 150 mM) bei pH 8.0 (5 mM Phosphat) im Vergleich zur Referenzextraktion (Ref., siehe auch Tabelle 2). Die OD-Werte wurde im kommerziell erhältlichen ELISA RIDASCREEN® Ochratoxin A 30/15 ermittelt.

| | Ref. | 70 mM | 60 mM | 50 mM | 40 mM | 30 mM | 20 mM |
|---|---|---|---|---|---|---|---|
| $OD_{Blank}$ | 1,952 | 1,213 | 1,421 | 1,457 | 1,474 | 1,555 | 1,647 |
| $OD_{Positiv}$ | 0,441 | 0,327 | 0,430 | 0,395 | 0,494 | 0,452 | 0,409 |
| Signalreduktion [%] | 77 | 73 | 70 | 73 | 67 | 71 | 75 |

Beispiel 20

Tabelle 23: Extraktionsausbeute von Ochratoxin aus Mais unter kombiniertem Einsatz der beanspruchten Substanzen 2,6-Naphthyldisulfonsäure (2,6-NDS, 125 mM) und 4-Hydroxyphenylsulfonsäure (4-OH-PSN, 50 mM und 75 mM) bei verschiedenen pH-Werten von 7.5 bis 9.0 im Vergleich zur Referenzextraktion (Ref., siehe auch Tabelle 2). Die OD-Werte wurde im kommerziell erhältlichen ELISA RIDASCREEN® Ochratoxin A 30/15 ermittelt.

| | | 125 mM 2,6-NDS | | | | | | | |
|---|---|---|---|---|---|---|---|---|---|
| | | 50 mM 4-OH-PSN | | | | 75 mM 4-OH-PSN | | | |
| | Ref. | pH 7.5 | pH 8.0 | pH 8.5 | pH 9.0 | pH 7.5 | pH 8.0 | pH 8.5 | pH 9.0 |
| $OD_{Blank}$ | 1,986 | 1,698 | 1,608 | 1,558 | 1,477 | 1,562 | 1,479 | 1,405 | 1,416 |
| $OD_{Positiv}$ | 0,484 | 0,408 | 0,292 | 0,358 | 0,266 | 0,424 | 0,342 | 0,281 | 0,329 |
| Signalreduktion [%] | 76 | 76 | 82 | 77 | 82 | 73 | 77 | 80 | 77 |

**Legende zu den Figuren**

Figur 1: Liste von in der Lebens- und Futtermittelindustrie relevanten Mykotoxinen. Neben der Auflistung sind auch deren chemische Strukturformeln und ihre Lösungseigenschaften in wässriger Umgebung dargestellt

## Patentansprüche

1. Verfahren zur Extraktion von Mykotoxinen aus Getreiden und anderen Lebensmitteln oder Futtermitteln, **dadurch gekennzeichnet, dass** wässrige, gepufferte Lösungen von Naphthyl- und/oder Phenylverbindungen und/oder deren heterozyklische Analoga mit den Getreiden oder anderen Lebensmitteln oder Futtermitteln in Kontakt gebracht werden, die wässrige Lösung anschließend abgetrennt und der Gehalt der extrahierten Mykotoxine in der wässrigen Lösung bestimmt wird und wobei als Naphthyl- und/oder Phenylverbindungen und/oder deren heterozyklische Analoga Verbindungen
der allgemeinen Formel I eingesetzt werden,

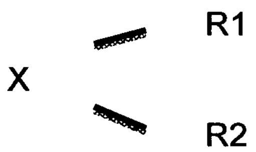

(I)

in der X, R1 und R2 folgende Bedeutung haben

X = Naphthyl- oder Phenylrest oder deren heterozyklische Analoga
R1 = wenigstens eine Sulfonsäuregruppe oder wenigstens eine Carbonsäuregruppe
R2 = unsubstituiert oder eine funktionelle Gruppe ausgewählt aus Hydroxy-, Alkyl-, Alkoxy-, Amino-, Sulfhydryl-, Halogen- und Thioether, die in o-, m- oder p-Stellung zur Säuregruppe im Molekül angeordnet sind.

2. Verfahren nach Anspruch 1, **dadurch gekennzeichnet, dass** eine oder mehrere der Naphthyl- und/oder Phenylverbindungen und/oder deren heterozyklische Analoga eingesetzt werden.

3. Verfahren nach einem der Ansprüche 1-2, **dadurch gekennzeichnet, dass** Verbindungen mit X = Naphthyl, R1 = Disulfonsäure oder X = Phenyl, R1 = Sulfonsäure und R2 = Hydroxy eingesetzt werden.

4. Verfahren nach einem der Ansprüche 1-3, **dadurch gekennzeichnet, dass** 1,5-Naphtyldisulfonsäure, 2,6-Naphtyldisulfonsäure und/oder Hydroxyphenylsulfonsäure eingesetzt werden.

5. Verfahren nach einem der Ansprüche 1-4, **dadurch gekennzeichnet, dass** die eingesetzten Naphthyl- und/oder Phenylverbindungen und/oder deren heterozyklische Analoga in Konzentrationen von 5 bis 600 mM in Abhängigkeit von deren maximalen Löslichkeit eingesetzt werden.

6. Verfahren nach einem der Ansprüche 1-5, **dadurch gekennzeichnet, dass** die Naphthyl- und/oder Phenylverbindungen und/oder deren heterozyklische Analoga als Lösung, in Pulver- oder in Tablettenform appliziert werden.

7. Verfahren nach einem der Ansprüche 1-6, **dadurch gekennzeichnet, dass** die wässrige Lösung von Naphthyl- und/oder Phenylverbindungen und/oder deren heterozyklische Analoga im Bereich von pH 5 - 10 gepuffert vorliegt.

8. Verfahren nach einem der Ansprüche 1-7, **dadurch gekennzeichnet, dass** der Gehalt an extrahierten Mykotoxinen in Antikörper-gestützten Systemen wie z.B. ELISA oder Lateral-Flow-Systemen bestimmt wird.

9. Verfahren nach einem der Ansprüche 1-8, **dadurch gekennzeichnet, dass** der Gehalt an extrahierten Mykotoxinen in chromatographisch-gestützten Systemen bestimmt wird.

10. Verfahren nach einem der Ansprüche 1-9, **dadurch gekennzeichnet, dass** die extrahierten Mykotoxine einer weiteren Reinigung mittels Immunoaffinitätschromatographiesäulen unterworfen werden.

11. Verwendung des Verfahrens nach einem der Ansprüche 1-10 zur Entfernung von Aflatoxin B1, Aflatoxin B2, Aflatoxin G1, Aflatoxin G2, Aflatoxin M1, Aflatoxin M2, Fumonisin B1, Fumonisin B2, Fumonisin B3, Deoxynivalenol, Ochratoxin A, Zearalenon, T-2, HT-2, Citrinin, Sterigmatocystein und Ergotalkaloiden.

## Claims

1. A method for extracting mycotoxins from grain and other foodstuffs or animal feed, **characterized in that** aqueous, buffered solutions of naphthyl and/or phenyl compounds and/or their heterocyclical analogues are brought into contact with the grain or other foodstuffs or animal feed, the aqueous solution is then separated out and the content of the extracted mycotoxins in the aqueous solution is determined, and whereby as naphthyl and/or phenyl compounds and/or their heterocyclical analogues, compounds with the general formula I are used.

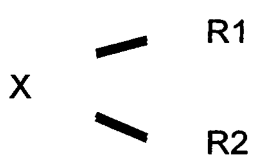

in which X, R1 and R2 have the following meaning

X = naphthyl or phenyl residue or its heterocyclical analogues
R1 = at least one sulphonic acid group or at least one carbonic acid group
R2 = unsubstituted or selected from a functional group of hydroxy, alkyl, alkoxy, amino, sulfhydryl, halogen and thioether, which are arranged in the o, m or p position in relation to the acid group in the molecule.

2. The method according to claim 1, **characterized in that** one or more of the naphthyl and/or phenyl compounds and/or their heterocyclical analogues are used.

3. The method according to one of claims 1-2, **characterized in that** the compounds with X = naphthyl, R1 = disulphonic acid or X = phenyl, R1= sulphonic acid and R2 = hydroxy are used.

4. The method according to any one of claims 1-3, **characterized in that** 1,5-naphthyl sulphonic acid, 2,6-naphthyl disulphonic acid and/or hydroxyphenyl sulphonic acid are used.

5. The method according to any one of claims 1-4, **characterized in that** the naphthyl and/or phenyl compounds used and/or their heterocyclical analogues are used in concentrations of 5 to 600 mM depending on their maximum solubility.

6. The method according to any one of claims 1-5, **characterized in that** the naphthyl and/or phenyl compounds and/or their heterocyclical analogues are applied as a solution, in powder or in tablet form.

7. The method according to any one of claims 1-6, **characterized in that** the aqueous solution of naphthyl and/or phenyl compounds and/or their heterocyclical analogues are buffered in the range of pH 5 - 10.

8. The method according to any one of claims 1-7, **characterized in that** the content of the extracted mycotoxins in antibody-supported systems such as ELISA or lateral flow systems is determined.

9. The method according to any one of claims 1-8, **characterized in that** the content of the extracted mycotoxins in chromatograph-supported systems is determined.

10. The method according to any one of claims 1-9, **characterized in that** the extracted mycotoxins are subjected to further cleaning using immunoaffinity chromatography columns.

11. The use of the method according to any one of claims 1-10 for removing aflatoxin B1, aflatoxin B2, aflatoxin G1, aflatoxin G2, aflatoxin M1, aflatoxin M2, fumonisin B1, fumonisin B2, fumonisin B3, deoxynivalenol, ochratoxin A, zearalenone, T-2, HT-2, citrinin, sterigmatocystin and ergot alkaloids.

**Revendications**

1. Procédé pour l'extraction de mycotoxines de céréales et d'autres denrées alimentaires ou aliments pour animaux, **caractérisé par le fait que** des solutions aqueuses tamponnées de composés naphtyliques et/ou phényliques et/ou de leurs analogues hétérocycliques sont mises en contact avec les céréales ou d'autres denrées alimentaires ou aliments pour animaux, que la solution aqueuse est ensuite séparée et que la teneur des mycotoxines extraites dans la solution aqueuse est déterminée et, comme composés naphtyliques et/ou phényliques et/ou leurs analogues hétérocycliques, des composés de la formule générale étant utilisés,

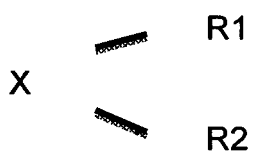

X, R1 et R2 ayant la signification suivante

X = radical naphtyle ou phényle ou leurs analogues hétérocycliques
R1 = au moins un groupement acide sulfonique ou au moins un groupement acide carboxylique
R2 = non substitué ou un groupe fonctionnel sélectionné à partir d'hydroxy-éther, d'alkyléther, d'alcoxyéther, d'aminoéther, d'éther de sulfhydryle, d'éther halogéné et de thioéther, disposés en position o, m ou p pour le groupe acide dans la molécule.

2. Procédé conformément à la revendication n°1, **caractérisé par le fait qu'**un ou plusieurs des composés naphtyliques et/ou phényliques et/ou de leurs analogues hétérocycliques sont utilisés.

3. Procédé conformément à l'une des revendications n°1 à n°2, **caractérisé par le fait que** des composés avec X = naphtyle, R1 = acide disulfonique ou X = phényle, R1 = acide sulfonique et R2 = hydroxy sont utilisés.

4. Procédé conformément à l'une des revendications n°1 à n°3, **caractérisé par le fait que** de l'acide disulfonique de naphtyle 1,5, de l'acide disulfonique de naphtyle 2,6 et/ou de l'acide sulfonique d'hydroxyphényle sont utilisés.

5. Procédé conformément à l'une des revendications n°1 à n°4, **caractérisé par le fait que** les composés naphtyliques et/ou phényliques mis en oeuvre et/ou leurs analogues hétérocycliques sont utilisés dans des concentrations de 5 à 600 mM en fonction de leur solubilité maximale.

6. Procédé conformément à l'une des revendications n°1 à n°5, **caractérisé par le fait que** les composés naphtyliques et/ou phényliques et/ou leurs analogues hétérocycliques sont appliqués comme solution sous forme de poudre ou de comprimés.

7. Procédé conformément à l'une des revendications n°1 à n°6, **caractérisé par le fait que** la solution aqueuse de composés naphtyliques et/ou phényliques et/ou de leurs analogues hétérocycliques est présente sous forme tamponnée dans la plage de pH de 5 à 10.

8. Procédé conformément à l'une des revendications n°1 à n°7, **caractérisé par le fait que** la teneur en mycotoxines extraites est déterminée dans des systèmes utilisant des anticorps, comme les systèmes ELISA ou Lateral-Flow.

9. Procédé conformément à l'une des revendications n°1 à n°8, **caractérisé par le fait que** la teneur en mycotoxines extraites est déterminée dans des systèmes assistés par chromatographie.

10. Procédé conformément à l'une des revendications n°1 à n°9, **caractérisé par le fait que** les mycotoxines extraites sont soumises à une purification supplémentaire au moyen de colonnes de chromatographie d'immunoaffinité.

11. Utilisation du procédé conformément à l'une des revendications n°1 à n°10 pour l'élimination de l'aflatoxine B1, l'aflatoxine B2, l'aflatoxine G1, l'aflatoxine G2, l'aflatoxine M1, l'aflatoxine M2, la fumonisine B1, la fumonisine B2, la fumonisine B3, le déoxynivalénol, l'ochratoxine A, le zéaralénone, le T-2, le HT-2, la citrinine, la stérigmatocystine et les alcaloïdes de l'ergot.

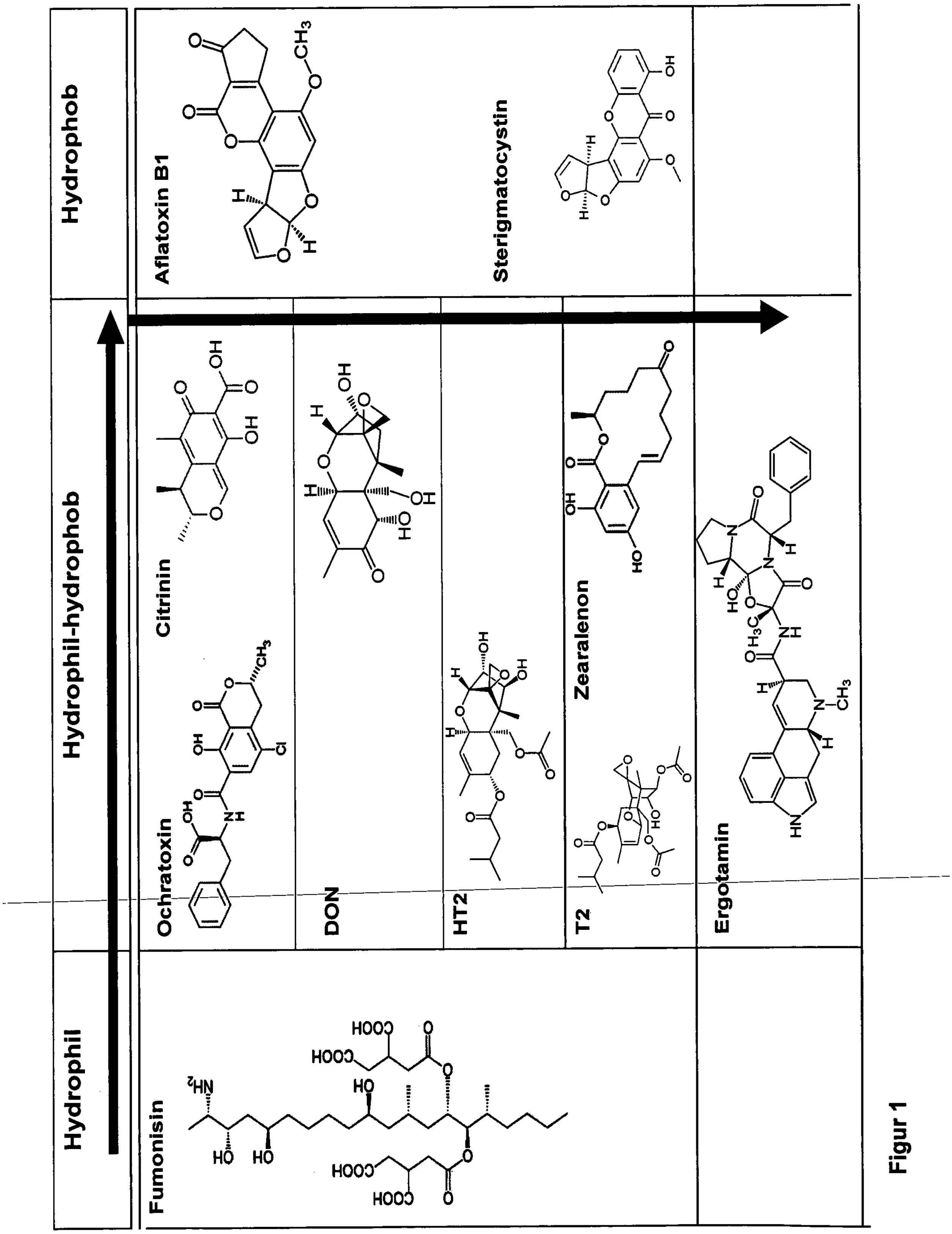
Hydrophil
Hydrophil-hydrophob
Hydrophob
Fumonisin
Ochratoxin
Citrinin
DON
HT2
T2
Zearalenon
Ergotamin
Aflatoxin B1
Sterigmatocystin

Figur 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20140356978 A
- WO 2015188205 A
- WO 2016057044 A


### In der Beschreibung aufgeführte Nicht-Patentliteratur


- **MISHRA et al.** *Food Add.Contam.,* 2016, vol. 33, 500-508